# EUROPÄISCHE PATENTSCHRIFT

(11) **EP 4 251 597 B1**
(45) Veröffentlichungstag und Bekanntmachung des Hinweises auf die Patenterteilung: **31.07.2024**
(21) Anmeldenummer: 22702900.6
(22) Anmeldetag: 21.01.2022
(51) Int. Cl.: C07C 7/08, C07C 9/12, C07C 11/08

(54) **ENERGIEEFFIZIENTES VERFAHREN ZUR ABTRENNUNG VON BUTENEN AUS C4-KOHLENWASSERSTOFFSTRÖMEN UND ANSCHLIESSENDER N/ISO-TRENNUNG**
ENERGY-EFFICIENT METHOD FOR SEPARATING BUTENES FROM C4 HYDROCARBON FLOWS AND SUBSEQUENT N/ISO SEPARATION
PROCÉDÉ À EFFICACITÉ ÉNERGÉTIQUE DESTINÉ À LA SÉPARATION DES BUTÈNES DES FLUX D'HYDROCARBURE C4 ET SÉPARATION N/ISO ULTÉRIEURE

(30) Priorität: 27.01.2021 EP 21153667
(43) Veröffentlichungstag der Anmeldung: 04.10.2023
(73) Patentinhaber: Evonik Oxeno GmbH & Co. KG, 45772 Marl (DE)
(72) Erfinder: LUTZE, Philip, 46535 Dinslaken (DE); PEITZ, Stephan, 45739 Oer-Erkenschwick (DE); RIX, Armin Matthias, 45770 Marl (DE); SIX, Tanita Valèrie, 44309 Dortmund (DE); SCHRÖDER, Moritz, 48153 Münster (DE); PAUL, Niklas, 45770 Marl (DE)
(74) Vertreter: Evonik Patent Association
(86) Internationale Anmeldenummer: PCT/EP2022/051387
(87) Internationale Veröffentlichungsnummer: WO 2022/161874

(56) Entgegenhaltungen:
- US-A- 4 556 461
- US-A1- 2014 124 358

## Beschreibung

Die vorliegende Erfindung betrifft ein Verfahren zur Abtrennung von Butenen aus C4-Kohlenwasserstoffströmen, die neben den Butenen auch Butane enthält, durch Extraktivdestillation mit einem geeigneten Lösemittel. Das erfindungsgemäße Verfahren zeichnet sich durch eine Wärmeintegration aus, mit der die Wärme des Lösemittels zur Erwärmung und/oder zumindest teilweiser Verdampfung verschiedener Ströme genutzt wird.

Die Trennung von Butan-Buten-Gemischen mittels Extraktivdestillation ist an sich bekannt. Dabei wird ein aprotisches Lösemittel (z. B. N-Methyl-2-pyrrolidon (NMP) oder Acetonitril (ACN)) eingesetzt, um die relative Flüchtigkeit der Alkane gegenüber den Alkenen zu erhöhen. In einer Extraktivdestillationskolonne, dem Absorber, werden bevorzugt die Butene im Lösemittel gelöst, die Butane werden als Kopfprodukt abgetrennt. Das beladene Lösemittel wird anschließend in einer Stripkolonne, dem Desorber, bei erhöhter Temperatur und/oder reduziertem Druck von den Butenen befreit, die als Kopfprodukt in angereicherter Form gewonnen werden. Das von Butenen befreite Lösemittel wird dann zur Extraktivdestillation zurückgefahren.

Aufgrund des hohen Lösemittel-zu-Feed-Verhältnisses kommt der Wärmeintegration eine hohe Bedeutung für die Wirtschaftlichkeit des Verfahrens zu. Im Sumpf des Desorbers fällt heißes Lösemittel an, dessen Energieinhalt auf verschiedene Weise genutzt werden kann. In der US 2014/0124358 A1 wird ein Verfahren zur selektiven Extraktion von Olefinen vorgestellt, welches die Aufgabe der Wärmeintegration lösen soll. Darin wird vorgeschlagen, den Energiegehalt des heißen Lösemittel zur Erwärmung eines Seitenstroms aus dem Desorber, zur Erwärmung des Sumpfprodukts des Absorbers, das zum Desorber geleitet wird, zur Erwärmung von einem oder mehreren Seitenströmen des Absorbers, und zur Vorwärmung des Feedstroms zu nutzen.

Die im Stand der Technik vorgeschlagene Lösung kann die Aufgabe einer möglichst vollständigen Rückgewinnung der im System vorhandenen Energieströme jedoch entweder nicht ganz oder nur durch relativ komplexe Aufbauten erfüllen.

Die Aufgabe der vorliegenden Erfindung bestand deshalb darin ein Verfahren bereitzustellen, bei dem eine verbesserte, bestenfalls maximale Energierückgewinnung erzielt wird und das anlagentechnisch weniger aufwändig ist.

Diese Aufgabe kann durch die in Anspruch 1 vorgeschlagene Ausführung des Verfahrens gelöst werden. Bevorzugte Ausführungsformen sind in den Unteransprüchen angegeben. Das erfindungsgemäße Verfahren ist ein Verfahren zur Abtrennung von Butenen aus einem C4-Kohlenwasserstoffstrom, der zumindest Butene und die Butane n-Butan und iso-Butan enthält, durch Extraktivdestillation mit einem Lösemittel, wobei das Verfahren die folgenden Schritte umfasst:
a. Zumindest teilweises Verdampfen des flüssigen C4-Kohlenwasserstoffstroms in einem Feedverdampfer, Zuführen des gasförmigen C4-Kohlenwasserstoffstroms und Zuführen des flüssigen Lösemittels, vorzugsweise NMP, oberhalb des C4-Kohlenwasserstoffstroms zu einem Absorber, in dem der C4-Kohlenwasserstoffstrom und das Lösemittel miteinander in Kontakt gebracht werden, wodurch überwiegend Butene aus dem C4-Kohlenwasserstoffstrom in das Lösemittel übergehen, wobei das so beladene Lösemittel, vorzugsweise NMP, in einem Flüssigkeitssammler des Absorbers gesammelt, durch einen Absorberverdampfer gefahren und dann unterhalb des Flüssigkeitssammlers in den Sumpf des Absorbers geleitet wird, wodurch überwiegend Butane aus dem beladenen Lösemittel, vorzugsweise NMP, ausgegast werden, wobei das beladene Lösemittel, vorzugsweise NMP, anschließend als Sumpfstrom zu einem Desorber geführt wird und wobei am Kopf des Absorbers ein im Vergleich zum eingesetzten C4-Kohlenwasserstoffstrom an Butanen angereicherter Strom anfällt;
b. Zuführen des beladenen Lösemittels, vorzugsweise NMP, zu dem Desorber, in dessen Sumpf im Vergleich mit dem Sumpf des Absorber eine erhöhte Temperatur und bevorzugt ein geringerer Druck vorliegt und in dem die Butene vom Lösemittel, vorzugsweise NMP, getrennt werden, wodurch am Kopf des Desorbers ein an Butenen angereicherter Strom anfällt, wobei ein zumindest teilweise von Butenen befreite Lösemittel vorzugsweise NMP, in einem Flüssigkeitssammler des Desorbers gesammelt, durch einen Desorberverdampfer gefahren und dann unterhalb des Flüssigkeitssammlers in den Sumpf des Desorbers geleitet wird, wodurch noch im Lösemittel vorhandene Butene ausgegast werden, und wobei das Lösemittel anschließend als Sumpfstrom zum Absorber zurückgeführt wird;
c. Zuführen des an Butanen angereicherten Stroms zu einem n/iso-Splitter, in dem n-Butan und iso-Butan voneinander getrennt werden, wodurch am Kopf des n/iso-Splitters ein an iso-Butanen angereicherter Strom und im Sumpf des n/iso-Splitters ein an n-Butanen angereicherter Strom anfällt;

wobei die Wärme des als Sumpfstrom des Desorbers abgenommen Lösemittels zur Wärmeintegration verwendet wird, in dem die Wärme des Lösemittels in jeweils mindestens einem Wärmetauscher für die Vorwärmung des zum Desorber geführten beladenen Lösemittels, zur Verdampfung im Absorberverdampfer und zur Verdampfung des flüssigen C4-Kohlenwasserstoffstroms eingesetzt wird, und wobei
der an Butenen angereichter Strom am Kopf des Desorbers entnommen und einer Kondensation unterworfen wird, wobei die Kondensationswärme zumindest teilweise zur Beheizung des n/iso-Splitters verwendet wird.

Ein Vorteil des vorliegenden Verfahrens ist der relativ einfache Aufbau der Wärmeintegration, die dennoch eine effiziente Energierückgewinnung ermöglicht. In der einfachsten Ausführungsform der vorliegenden Erfindung sind zudem nicht zwingend zusätzlichen Seitenverdampfer vorgesehen, die einen zusätzlichen anlagentechnischen Aufwand und damit höhere Kosten nach sich ziehen. Im vorliegenden Fall kann darüber hinaus die Kondensationswärme, die bei der Kondensation des an Butenen angereicherten Strom entsteht, und optional die Hydrierwärme von einer vorgeschalteten oder einer oder zwei nachgeschalteten Hydrierungsschritten für eine n/iso-Trennung der Butane mittels eines n/iso-Splitters genutzt werden, wodurch weniger oder gar keine externe Energie für einen solchen Schritt zugeführt werden muss. Auch in dem erfindungsgemäßen Verfahren oder anderen zusätzlichen Verbundverfahren anfallende Abwärmen wären für die Beheizung des n/iso-Splitters einsetzbar.

Durch die Wärmeintegration wird dem Lösemittel Wärme entzogen. Der Grund dafür ist nicht nur, dass damit andere Ströme oder Kolonnen geheizt werden sollen, sondern in erster Linie die Abkühlung des Lösemittels für die Absorption. Die Absorption der Butene (hier: Schritt a) findet meistens bei einer geringeren Temperatur statt als die Desorption (hier: Schritt b). Wird dem Lösemittel bei der Wärmeintegration ausreichend Wärme entzogen, weist also eine geeignete Temperatur auf, kann das Lösemittel direkt in den Absorber gefahren werden. Es ist jedoch auch denkbar, dass das Lösemittel trotz der vorliegenden Wärmeintegration noch nicht die richtige Temperatur aufweist. In so einem Fall kann das Lösemittel nach der Wärmeintegration und vor dem Eintritt in den Absorber durch einen Restkühler geleitet werden, um auf eine geeignete Temperatur gekühlt zu werden.

Wärme ist eine Prozessgröße. Die zu- oder abgeführte Wärme entspricht der Änderung der Inneren Energie abzüglich der verrichteten Arbeit. Bei den in dieser Erfindung verwendeten Begriffe Wärme, Wärmetransport und Wärmeintegration wird diese Definition stets zu Grunde gelegt.

Das vorliegende Verfahren betrifft die Abtrennung von Butenen aus butenhaltigen C4-Kohlenwasserstoffströmen. Diese Ströme enthalten neben den Butenen auch Alkane (n-Butane und iso-Butane). Der Begriff Butane wird im Rahmen der vorliegenden Erfindung dabei so verstanden, dass er - sofern nichts anderes bezeichnet ist - sowohl n-Butan als auch Isobutan meint. Im vorliegenden Verfahren soll nach der zumindest teilweisen Abtrennung der Butene noch eine Auftrennung der Alkane in n-Butan und iso-Butan stattfinden, um n-Butan und iso-Butan jeweils möglichst rein zu erhalten. Dazu ist erfindungsgemäß eine n/iso-Trennung in einem n/iso-Splitter vorgesehen. Vor oder nach dieser Auftrennung der Butane kann eine Hydrierung der Restbutene in den Strömen durchgeführt werden. Im erfindungsgemäßen Verfahren können daher alle C4-Kohlenwasserstoffströme eingesetzt werden, die zumindest Butene und Butane enthalten, sofern die Mengen, in denen die Butene und/oder Butane vorhanden sind, eine wirtschaftliche Durchführung des Verfahrens zulassen. In einer bevorzugten Ausführungsform der vorliegenden Erfindung besteht der eingesetzte C4-Kohlenwasserstoffstrom im Wesentlichen, d.h. zu mehr als 98 Gew.-%, vorzugsweise zu mehr als 99 Gew.-% aus Butanen und Butenen. Die entsprechenden Ströme können auch Verunreinigungen oder andere Kohlenwasserstoffe, wie 1,3-Butadien oder C5-Kohlenwasserstoffe, in geringen Mengen enthalten.

In dem erfindungsgemäßen Verfahren wird in Schritt a ein flüssiges Lösemittel eingesetzt, in dem sich vorrangig die Butene des eingesetzten, gasförmigen C4-Kohlenwasserstoffstroms lösen. Geeignete Lösemittel sind aprotische Lösemittel, beispielsweise N-Methyl-2-pyrrolidon (NMP). Das erfindungsgemäße Verfahren wird vorzugsweise mit NMP als Lösemittel durchgeführt. In einer weiterhin bevorzugten Ausführungsform der vorliegenden Erfindung enthält das Lösemittel Wasser, insbesondere im Bereich von 1 bis 10 Gew.-%, vorzugsweise von 4 bis 9 Gew.-%, jeweils bezogen auf die Gesamtmenge an Lösemittel.

Als Absorber können insbesondere Füllkörperkolonnen dienen, die mindestens zwei Füllkörperbetten aufweisen. Solche Kolonnen sind dem Fachmann grundsätzlich bekannt. Oberhalb des ersten Füllkörperbettes befindet sich vorzugsweise eine Rückwaschzone mit mehreren theoretischen Böden, um das in die Gasphase mitgerissene Lösemittel zurückzuhalten. Oberhalb der Rückwaschzone ist der Kopf des Absorbers, wo ein im Vergleich zum eingesetzten C4-Kohlenwasserstoffstrom an Butanen angereicherter Strom anfällt. Unter dem letzten Füllkörperbett würde der erfindungsgemäße Flüssigkeitssammler angeordnet sein, worunter sich der Sumpf des Absorbers befindet. Der exakte Aufbau des Absorbers hängt von verschiedenen Parametern ab und ist in gewisser Hinsicht variabel.

Das flüssige Lösemittel wird räumlich betrachtet oberhalb des Einlasses für den C4-Kohlenwasserstoffstroms zu dem Absorber gegeben. In einer bevorzugten Ausführungsform wird das Lösemittel oberhalb des ersten Füllkörperbettes und der C4-Kohlenwasserstoffstrom in ein oder mehrere Füllkörperbetten unterhalb des ersten Füllkörperbettes zum Absorber gegeben. Das flüssige Lösemittel wird im Absorber nach unten rieseln und mit dem (aufsteigenden) dampfförmigen C4-Kohlenwasserstoffstrom in Kontakt gebracht, wodurch ein Teil des C4-Kohlenwasserstoffstroms, der überwiegend Butene enthält, in das Lösemittel übergeht, wodurch ein beladenes Lösemittel entsteht. Der C4-Kohlenwasserstoffstrom und das Lösemittel werden in Schritt a insbesondere im Gegenstrom miteinander in Kontakt gebracht. In einer bevorzugten Ausführungsform der vorliegenden Erfindung umfasst der Teil des C4-Kohlenwasserstoffstroms, der in das Lösemittel übergeht, mindestens 70 Gew.-%, besonders bevorzugt mindestens 80 Gew.-% Butene, bezogen auf die Zusammensetzung des in das Lösemittel übergegangenen Teils des C4-Kohlenwasserstoffstroms. Daraus ergibt sich insbesondere, dass mindestens 80%, besonders bevorzugt mindestens 90% der im eingesetzten C4-Kohlenwasserstoffstrom enthaltenen Butene in das Lösemittel übergehen.

Das beladene Lösemittel läuft im Absorber nach unten und wird in einem geeigneten Flüssigkeitssammler, insbesondere einem Kaminboden, gesammelt. Die Temperatur des im Flüssigkeitssammler anfallenden beladenen Lösemittels beträgt vorzugsweise zwischen 40 und 90°C, besonders bevorzugt zwischen 45 und 65 °C. Vom Flüssigkeitssammler wird das beladene Lösemittel abgenommen, durch einen Absorberverdampfer gefahren und dann unterhalb des Flüssigkeitssammlers in den Sumpf des Absorbers geleitet, wodurch überwiegend Butane aus dem beladenen Lösemittel ausgegast werden. Der Absorberverdampfer ist vorzugsweise ein Once-Through-Verdampfer, bei dem das beladene Lösemittel nur einmal durch den Verdampfer geleitet wird. Dadurch lassen sich möglichst niedrige Temperaturen realisieren, wodurch Fouling verhindert werden kann. Zudem wird die treibende Temperaturdifferenz vergrößert, was eine noch effizientere Energieausnutzung des NMP-Stroms ermöglicht. Der Absorberverdampfer kann auch mehrstufig ausgestaltet sein, d. h. es können mehrere Wärmetauscher bzw. mehrere Verdampfer vorhanden sein, die zum Absorberverdampfer gehören.

Im Sumpf verbleibt dann das vorwiegend mit Butenen beladene Lösemittel, welches von dort abgenommen und als Sumpfstrom zum Desorber geführt wird. Die Temperatur im Sumpfstrom des Absorbers, der zum Desorber geführt wird, beträgt dabei vorzugsweise zwischen 70 und 130 °C, besonders bevorzugt zwischen 85 und 120 °C.

Am Kopf des Absorbers fällt ein im Vergleich zum eingesetzten C4-Kohlenwasserstoffstrom an Butanen angereicherter Strom an. Dieser an Butanen angereicherte Strom kann zusätzlich Wasser enthalten, welches aus dem Lösemittel stammt. Dieses Wasser kann in einem nachfolgenden Schritt abgetrennt werden. Dabei wird der an Butanen angereicherte Strom am Kopf des Absorbers entnommen und einer ein- oder mehrstufigen Kondensation unterworfen, wobei ein wasserhaltiger Strom und ein butanhaltiger Produktstrom auskondensiert werden. Diese beiden Ströme können in einer geeigneten Vorrichtung, beispielsweise einem Euter, voneinander getrennt werden. Der vom butanhaltigen Produktstrom abgetrennte wasserhaltige Strom kann je nach seiner Zusammensetzung zum Absorber oder zum Desorber geführt und/oder teilweise aus dem Verfahren ausgeschleust werden.

Der so aus der Kondensation erhaltene butanhaltige Produktstrom kann noch geringe Mengen an Wasser enthalten, insbesondere in einer Menge von bis zu 1500 Gew.-ppm, bezogen auf die Gesamtzusammensetzung des butanhaltigen Produktstroms. Außerdem kann der aus der Kondensation erhaltene butanhaltige Produktstrom noch Restbutene enthalten, wobei die Ströme üblicherweise weniger als 20 Gew.-%, vorzugsweise weniger als 15 Gew.-%, besonders bevorzugt weniger als 5 Gew.-% an Butenen, bezogen auf die Gesamtzusammensetzung des butanhaltigen Produktstroms, enthalten.

Je nach den Anforderungen an den erhaltenen butanhaltigen Produktstrom kann es erforderlich sein, dass der butanhaltige Produktstrom nach der Kondensation einer Trocknung, vorzugsweise in einer Trocknungskolonne, unterworfen wird, um das noch enthaltene Wasser abzutrennen. Vorzugsweise weist der butanhaltige Produktstrom nach der Trocknung eine maximale Menge an Wasser von 50 Gew.-ppm, vorzugsweise von 25 Gew.-ppm auf. Das bei der Trocknung anfallende Wasser kann zur Kondensation am Absorber zurückgeführt werden.

Erfindungsgemäß wird der an Butanen angereicherte Strom oder, sofern eine Kondensation erfolgt, der butanhaltige Produktstrom zu einem n/iso-Splitter geführt, in dem n-Butan und iso-Butan voneinander getrennt werden. Ein n/iso-Splitter ist insbesondere eine geeignete Destillationskolonne. Am Kopf des n/iso-Splitters fällt dabei ein an iso-Butan angereicherter Strom und im Sumpf des n/iso-Splitters ein an n-Butan angereicherter Strom an. Der erfindungsgemäße n/iso-Splitter ist eine zur Trennung der n- von den iso-Butanen geeignete Vorrichtung, insbesondere eine Destillationskolonne. Die Temperatur im Sumpf des n/iso-Splitters beträgt vorzugsweise 50 bis 70 °C, besonders bevorzugt 55 bis 65 °C. Der Druck im n/iso-Splitter beträgt vorzugsweise 4 bis 10 bar absolut, besonders bevorzugt 5 bis 7 bar absolut. Die exakte Ausgestaltung und der Betrieb eines entsprechenden n/iso-Splitters bzw. einer entsprechenden Destillationskolonne ist dem Fachmann bekannt.

Der am Kopf des n/iso-Splitters anfallende an iso-Butanen angereicherte Strom besteht überwiegend aus iso-Butan. Vorzugsweise besteht der an iso-Butan angereicherte Strom zu 98 Gew.-%, weiterhin bevorzugt zu 99 Gew.-%, besonders bevorzugt zu 99,5 Gew.-% aus iso-Butan. Der im Sumpf des n/iso-Splitters anfallende an n-Butanen angereicherte Strom besteht überwiegend aus n-Butan. Vorzugsweise besteht der an n-Butan angereicherte Strom zu 98 Gew.-%, weiterhin bevorzugt zu 99 Gew.-%, besonders bevorzugt zu 99,5 Gew.-% aus n-Butan.

Die zur Beheizung des n/iso-Splitters und damit zum Erreichen der Auftrennung der Butane notwendige Energie wird zumindest teilweise aus der Kondensationswärme erhalten, die bei der Kondensation des an Butenen angereicherten Stroms, der am Kopf des Desorbers entnommen wird, entsteht. Die Kondensationswärme wird somit zumindest teilweise genutzt. Der entsprechende Kondensator für die Kondensation des an Butenen angereicherten Stroms, der am Kopf des Desorbers entnommen wird, kann ein dem Fachmann bekannter Wärmetauscher sein. Denkbar ist auch der Kondensator des Desorbers als Kettle ausgeführt wird. Es ist auch möglich, dass der Kondensator, je nach Verfügbarkeit interner Ströme, mehrstufig ausgeführt wird, wobei eine Wärmeintegration nur mit der Kondensation in der ersten Stufe erfolgt und in der zweiten Stufen ein externes Kühlmedium verwendet wird.

Es ist bereits erwähnt worden, dass der an Butanen angereicherte Strom oder, sofern eine Kondensation erfolgt, der butanhaltige Produktstrom bereits zu überwiegendem Teil aus n- und iso-Butanen besteht. Möglich ist, dass auch Butene, die bei der Extraktion nicht in das Lösemittel übergegangen sind, noch in diesen Strömen vorhanden sind. Ist es gewünscht, dass mit der n/iso-Trennung olefinreine n- bzw. iso-Butan-Ströme erhalten werden, kann der an Butanen angereicherte Strom, der am Kopf des Absorbers abgenommen wird, oder, sofern eine Kondensation erfolgt, der butanhaltige Produktstrom vor dem Eintritt in den n/iso-Splitter einer Hydrierung unterworfen werden, um noch vorhandene Butene zu Butanen umzusetzen. Eine solche Restolefinhydrierung ist dem Fachmann allgemein geläufig und soll hier nicht detailliert beschrieben werden.

In einer besonders bevorzugten Ausführungsform wird zumindest ein Teil der bei der Hydrierung vor dem n/iso-Splitter entstehenden Reaktionswärme zur Beheizung des n/iso-Splitters verwendet. Dadurch kann zumindest ein Teil der sonst notwendigen externen Energie zur Beheizung des n/iso-Splitters eingespart werden.

Es ist alternativ auch möglich, dass eine solche Hydrierung nicht vor dem n/iso-Splitter angeordnet ist, sondern erst nachher. Üblicherweise fallen noch vorhandene 1-Butene und Isobutene am Kopf des n/iso-Splitters an und wären dann in dem an iso-Butan angereicherten Strom enthalten. Die 2-Butene landen im Sumpf und sind im an n-Butan angereicherten Strom enthalten. In einer bevorzugten Ausführungsform der vorliegenden Erfindung wird einer von dem an iso-Butan angereicherten Strom und dem an n-Butanen angereicherten Strom oder werden beide Ströme einer Hydrierung unterworfen, um noch vorhandene Butene zu Butanen umzusetzen. Es ist somit möglich, auch nur einen aus dem n/iso-Splitter erhaltenen Strom zu hydrieren, während der andere Strom nicht hydriert wird.

In einer besonders bevorzugten Ausführungsform der vorliegenden Erfindung wird zumindest ein Teil der bei der Hydrierung eines der beiden Ströme oder zumindest ein Teil der bei den beiden Hydrierungen der beiden Ströme entstehenden Reaktionswärme zur Beheizung des n/iso-Splitters verwendet. Dadurch kann zusätzliche der sonst notwendigen externen Energie zur Beheizung des n/iso-Splitters eingespart werden. Unter Umständen wird keine zusätzliche externe Energie mehr benötigt. Eine entsprechende Wärmeintegration der entstehenden Reaktionswärme könnte beispielsweise auch durch einen internen Warmwasserkreislauf geleistet werden. Dem Fachmann sind solche anlagentechnischen Schritte an sich geläufig.

Das im Sumpf des Absorbers abgenommene und vorwiegend mit Butenen beladenen Lösemittel wird dem Desorber zugeführt. Das beladene Lösemittel kann dazu beispielsweise mittels einer Pumpe zum Desorber geführt werden. Im Sumpf des Desorbers liegt im Vergleich zum Sumpf des Absorbers eine erhöhte Temperatur und bevorzugt ein geringerer Druck vor. Die Temperatur im Sumpf des Desorbers beträgt vorzugsweise zwischen 120 und 200 °C, weiterhin bevorzugt zwischen 130 und 195 °C. Der Kopfdruck im Desorber kann zwischen 1 und 6 bar absolut, vorzugsweise zwischen 2 und 5 bar absolut betragen. Durch die gegenüber dem Absorber erhöhte Temperatur und den vorzugsweise geringeren Druck werden die Butene und optional noch enthaltenen Butane zumindest teilweise aus dem Lösemittel entfernt. In einer bevorzugten Ausführungsform enthält das zumindest teilweise von Butenen befreite Lösemittel bis zu 5000 Gew.-ppm an Butenen, besonders bevorzugt 100 bis 900 Gew.-ppm an Butenen. Das zumindest teilweise von Butenen befreite Lösemittel läuft im Desorber nach unten und wird in einem Flüssigkeitssammler des Desorbers gesammelt. Von dort wird das zumindest teilweise von Butenen befreite Lösemittel durch einen Desorberverdampfer gefahren und dann unterhalb des Flüssigkeitssammlers, insbesondere einem Kaminboden, in den Sumpf des Desorbers geleitet, wodurch noch im Lösemittel vorhandene Butene ausgegast werden. Der Desorberverdampfer ist vorzugsweise ein Once-Through-Verdampfer, bei dem das zumindest teilweise von Butenen befreite Lösemittel nur einmal durch den Verdampfer geleitet wird. Dadurch lassen sich möglichst niedrige Temperaturen realisieren, wodurch Fouling verhindert werden kann. Der Desorberverdampfer kann auch mehrstufig ausgestaltet sein, d. h. es können mehrere Wärmetauscher vorhanden sein, die zum Desorberverdampfer gehören. Im Sumpf verbleibt dann das von Butenen befreite Lösemittel, welches von dort abgenommen, als Sumpfstrom zum Absorber geführt und dort wieder als Lösemittel für die Absorption von Butenen eingesetzt wird.

Das von Butenen befreite Lösemittel kann bevor es zum Absorber geführt wird teilweise oder vollständig einer Regeneration unterworfen werden, wodurch Verunreinigungen, beispielsweise die vorgenannt im eingesetzten C4-Kohlenwasserstoffstrom vorhandenen und/oder bei den Temperaturen im Desorber aus den Butenen gebildeten Nebenprodukte wie oligomere oder polymere Verbindungen, aus dem Lösemittel, vorzugsweise dem NMP, entfernt werden. Die Regeneration wird vorzugsweise so durchgeführt, dass das von Butenen befreite Lösemittel in einen Behälter gefahren und bei einem Druck von weniger als 500 mbar absolut, weiterhin bevorzugt von weniger als 200 mbar absolut und einer Temperatur zwischen 100 und 150 °C verdampft wird. An den Behälter kann eine Kolonne angeschlossen sein. Durch die Regeneration werden insbesondere Schwersieder abgetrennt. Wird nur ein Teil des von Butenen befreiten Lösemittels einer Regeneration unterworfen, wird der regenerierte Teil des Lösemittels anschließend mit dem nicht regenerierten Lösemittel vereint und zum Absorber zurückgeführt.

Am Kopf des Desorbers fällt dann insbesondere ein im Vergleich zu dem eingesetzten C4-Kohlenwasserstoffstrom an Butenen angereicherte Strom an. Dieser an Butenen angereicherte Strom kann zusätzlich Wasser enthalten, welches aus dem Lösemittel stammt. Dieses Wasser kann in einem nachfolgenden Schritt abgetrennt werden. Dabei wird der an Butenen angereichte Strom am Kopf des Desorbers entnommen und einer ein- oder mehrstufigen Kondensation unterworfen, wobei ein wasserhaltiger Strom, der neben Wasser auch noch Reste an Organik enthalten kann, und ein butenhaltiger Produktstrom auskondensiert werden. Diese beiden Ströme können in einer geeigneten Vorrichtung, beispielsweise einem Euter, voneinander getrennt werden. Der vom butenhaltigen Produktstrom abgetrennte wasserhaltige Strom kann anschließend zurück zum Desorber geführt werden. Möglich ist auch eine Ausschleusung des ganzen oder von Teilen des wasserhaltigen Stroms, um die Organik zu entfernen.

In einer bevorzugten Ausführungsform der vorliegenden Erfindung ist die Kondensation des am Kopf des Desorbers abgenommenen an Butenen angereicherten Stroms zweistufig ausgestaltet, wobei in einer ersten Stufe ein wasserhaltiger Strom auskondensiert wird, das dann zum Desorber zurückgeführt wird, und in der zweiten Stufe der butenhaltige Produktstrom auskondensiert wird. Es kann jedoch auch sein, dass in der zweiten Stufe auch noch vorhandenes Wasser auskondensiert. Dieses restliche Wasser kann über eine geeignete Vorrichtung, beispielsweise einen Euter, vom butenhaltigen Produktstrom abgetrennt werden.

Der aus der Kondensation erhaltene butenhaltige Produktstrom enthält vorzugsweise weniger als 20 Gew.-%, weiterhin bevorzugt weniger als 16 Gew.-% an Butanen bezogen auf die Gesamtzusammensetzung des butenhaltigen Produktstroms. Der aus der Kondensation erhaltene butenhaltige Produktstrom weist stattdessen vorzugsweise einen Butengehalt von mindestens 70 Gew.-%, weiterhin bevorzugt von mindestens 75 Gew.-%, besonders bevorzugt von mindestens 86 Gew.-% bezogen auf die Gesamtzusammensetzung des butenhaltigen Produktstroms auf.

Ein kennzeichnendes Merkmal der vorliegenden Erfindung ist die Wärmeintegration unter Verwendung der Wärme des Lösemittels auf dem Weg vom Desorber zurück zum Absorber. Die Wärme des als Sumpfstrom des Desorbers abgenommen Lösemittels, vorzugsweise des NMPs, wird erfindungsgemäß zur Wärmeintegration verwendet, in dem die Wärme des Lösemittels in jeweils mindestens einem Wärmetauscher für die Vorwärmung des zum Desorber geführten beladenen Lösemittels zur Verdampfung im Absorberverdampfer und zur Verdampfung des flüssigen C4-Kohlenwasserstoffstroms eingesetzt wird.

In einer bevorzugten Ausgestaltung der vorliegenden Erfindung wird die Vorwärmung des zum Desorber geführten beladenen Lösemittels zweistufig durchgeführt, wobei eine erste eine Wärmeübertragung auf das zum Desorber geführten beladenen Lösemittel in einem Wärmetauscher, beispielsweise einen Rohrbündelwärmetauscher, und eine zweite Wärmeübertragung auf das zum Desorber geführten beladenen Lösemittel in einem Kettleverdampfer erfolgt. Eine solche Ausgestaltung hat den Vorteil, dass bei der vorgenannten bevorzugten Ausgestaltung die Wärme, die in beiden Stufen, d. h. im Wärmetauscher und im Kettleverdampfer auf das beladene Lösemittel übertragen wird, aus dem als Sumpfstrom des Desorbers abgenommen Lösemittel als Wärmeträgermedium stammt. Die Verwendung eines Kettleverdampfers hätte auch den Vorteil, dass in der Leitung zum Desorber ein geringerer Vordruck herrschen könnte. Normalerweise ist ein hoher Vordruck notwendig, um das Verdampfen in der Rohrleitung zu verhindern, was zu Problemen bis zum Bersten der Rohrleitung führen könnte. Ein weiterer Vorteil ist, dass die Temperaturbelastung begrenzt und dadurch sichergestellt wird, dass die Temperaturdifferenz zur Wärmeübertragung ausreichend groß ist bzw. bleibt.

Erfindungsgemäß wird das zumindest teilweise von Butenen befreite Lösemittel, vorzugsweise NMP, in einem Flüssigkeitssammler des Desorbers gesammelt und durch einen Desorberverdampfer gefahren, wodurch noch im Lösemittel vorhandene Butene ausgegast werden können. Die Wärme zur Verdampfung im Desorberverdampfer kann dabei in einem Wärmetauscher durch Wärmeübertragung von einem geeigneten Wärmeträgermedium eingetragen werden. Das Wärmeträgermedium kann insbesondere Heizdampf sein, der als Mittel- oder Hochdruckdampf eingesetzt wird. Als Heizdampf ist ein Mitteldruckdampf bevorzugt, welcher eine Temperatur von 150 bis 270 °C, vorzugsweise von 160 bis 250 °C aufweist. Der Mitteldruckdampf weist vorzugsweise einen Druck von 15 bis 30 bar absolut, besonders bevorzugt von 17 bis 25 bar absolut auf. Als Heizdampf kann auch ein Dampf mit einem Druck von > 30 bar absolut eingesetzt werden. Ein solcher Heizdampf kann auch als Hochdruckdampf bezeichnet werden.

Der zur Verdampfung eingesetzte Heizdampf kann im Wärmetauscher zumindest teilweise kondensieren, wodurch ein Heißkondensat bei einem Druck von 10 bis 20 bar absolut, vorzugsweise 12 bis 17 bar absolut und einer Temperatur von 150 bis 210 °C, vorzugsweise 160 bis 200 °C anfällt. Nach dem Wärmetauscher ist vorzugsweise ein Kondensatbehälter angeordnet, in dem das Heißkondensat vom Dampf getrennt werden kann. Das Heißkondensat kann zusätzlich zur Beheizung des n/iso-Splitters verwendet werden. In dem Kondensatbehälter liegt vorzugsweise ein geringerer Druck vor als im Wärmetauscher auf der Heizdampfseite. Aufgrund des geringeren Drucks kann ein Teil des Heißkondensats verdampfen, wodurch der gesamte Dampf, d. h. der nicht kondensierte Anteil des Heizdampfes und das im Kondensatbehälter durch Druckentspannung verdampfte Heißkondensat, im Kondensatbehälter als Niederdruckdampf anfällt. Niederdruckdampf weist vorliegend vorzugsweise einen Druck von mehr als 0 bar und weniger als 10 bar absolut auf. Die Temperatur des Niederdruckdampfs beträgt vorzugsweise 100 bis 180 °C.

Der dort anfallende Niederdruckdampf enthält noch Energie, die in keinem bekannten Verfahren ausgenutzt wird. Aus energetischer und wirtschaftlicher Sicht ist das jedoch nicht sinnvoll. Diese Energie kann jedoch in einer bevorzugten Ausgestaltung der vorliegenden Erfindung genutzt werden. Dazu kann der zur Verdampfung im Desorberverdampfer eingesetzte Heizdampf mittels eines, vorzugsweise regelbaren Dampfstrahlers (Thermokompressors) zur Verfügung gestellt werden. Der Thermokompressor wird dann sowohl mit dem eingesetzten Heizdampf, der beispielsweise aus einem entsprechenden Dampfnetz stammt, hier insbesondere der bevorzugt eingesetzte Mitteldruckdampf, als auch mit dem Niederdruckdampf aus dem Kondensatbehälter gespeist, wodurch ein Mischdampf entsteht, der dementsprechend das Wärmeträgermedium für den Desorberverdampfer ist. Der Mischdampf ist in dieser Ausgestaltung demnach der Heizdampf. Ein solcher Dampfstrahler ist so ausgestaltet, dass er mit einem Treibdampf betrieben wird und durch einen Unterdruck (Staudruck im Dampfstrahler) Saugdampf aus einem Behälter ansaugen kann, wodurch dann der Mischdampf entsteht, der als Wärmeträgermedium eingesetzt wird. Der Treibdampf ist im vorliegenden Fall der Heizdampf bzw. der Mitteldruckdampf, mit dem der Niederdruckdampf als Saugdampf aus dem Kondensatbehälter angesaugt und mit dem Treibdampf vermischt wird.

Der Vorteil einer solchen Ausgestaltung ist offensichtlich. Die Energie des im Kondensatbehälter anfallenden Niederdruckdampfes kann genutzt und damit Energie und Kosten gespart werden. Eine solche Verfahrensweise kann auch aus einem anderen Grund vorteilhaft sein. Der eingesetzte Dampfstrahler kann dahingehend regelbar sein, dass die Mengen von Mitteldruck- und Niederdruckdampf, beispielsweise in Abhängigkeit von bestimmten Prozessparametern, eingestellt werden können. Die Saugdampfmenge stellt sich dabei über die Treibdampfmenge ein. Die Mengen von Mitteldruck- und Niederdruckdampf können beispielsweise in Abhängigkeit von der Temperatur im Desorber eingestellt werden.

Eine weiterhin bevorzugte Ausgestaltung kann auch dann vorliegen, wenn der Desorber einen Seitenverdampfer aufweist. In einem solchen Fall kann das Wärmeträgermedium, welches für den Seitenverdampfer eingesetzt wird, der Mischdampf aus dem Dampfstrahler sein, während im Desorberverdampfer Mitteldruckdampf als Heizdampf eingesetzt wird. Das Heißkondensat aus dem Desorberverdampfer und dem Seitenverdampfer werden dann entsprechend den obigen Ausführungen zu einem Kondensatbehälter geleitet. Der dort anfallende Niederdruckdampf wird dann im Dampfstrahler verwendet, dessen Mischdampf im Seitenverdampfer eingesetzt wird. Der Vorteil dieser Variante ist, dass das anfallende Heißkondensat weiter entspannt werden könnte, um eine größere Niederdruckdampfmenge bereitstellen zu können.

Das vorliegend beschriebene Verfahren kann in chemischen Verbünden, die insbesondere eine Oligomerisierung und optional eine Hydroformylierung umfassen, eingesetzt werden. Dabei ist es möglich, dass die Abtrennung von Butenen nach dem erfindungsgemäßen Verfahren an verschiedenen Stellen im Verbund eingesetzt wird. Es ist auch möglich, wenn die erfindungsgemäße Abtrennung von Butenen an mehreren Stellen innerhalb eines chemischen Verbundes vorhanden ist. Möglich ist beispielsweise, dass das hier beschriebene Verfahren zu Beginn eines solchen Verbunds eingesetzt wird. Der eingesetzte C4-Kohlenwasserstoffstrom kann dann insbesondere ein Crack-C4, ein Raffinat 1, ein Raffinat 2 oder eine Mischung daraus sein. Sofern Crack-C4 und/oder das Raffinat 2 eingesetzt werden, kann vor dem erfindungsgemäßen Abtrennverfahren eine Crack-C4-Hydrierung, in der Butadien selektiv hydriert wird, oder eine Butadienabtrennung, bei der Butadien extraktiv mit einem Lösemittel wie NMP oder Nitrilen entfernt wird, erfolgen, um den Gehalt an Butadien zu reduzieren. Nach einer extraktiven Butadienabtrennung und vor der erfindungsgemäßen Abtrennung kann eine Hydroisomerisierung angeordnet sein, um die Trennaufgabe beim erfindungsgemäßen Verfahren zu erleichtern, da 1-Buten in 2-Buten umgewandelt wird, was vom Lösemittel in der Regel besser aufgenommen wird.

Wird das Abtrennverfahren zu Beginn des Verbunds eingesetzt, kann der erhaltene Produktstrom einer MTBE-Synthese zugeführt werden und vorzugsweise anschließend sukzessive eine 1-Butenabtrennung, eine Oligomerisierung und eine oder mehrere Hydroformylierung(en) auf die aufgereinigten Oligomere erfolgen. Eine Hydroformylierung kann sowohl mit dem Produktstrom aus der Oligomerisierung erfolgen, wodurch zum Beispiel aus Di-n-butenen nach anschließender Hydrierung INA (Isononanol) oder aus Tributenen ITDA (Isotridecanal) hergestellt werden kann, als auch mit den nicht umgesetzten Butenen der Oligomerisierung, wodurch nach anschließender Aldolkondensation und nachfolgender Hydrierung 2-PH (2-Propylheptanol) hergestellt werden kann. Mit den nicht umgesetzten Butenen aus der Oligomerisierung könnte ggf. auch anstatt einer Hydroformylierung eine weitere Oligomerisierung betrieben werden. Die Bedingungen der einzelnen Verfahrensschritte sind dem Fachmann geläufig. Die einzelnen Verfahrensschritte können weitere Schritte wie beispielsweise die Abtrennung der Produkte oder die Aufarbeitung der resultierenden Ströme enthalten, sind hier jedoch nicht explizit genannt. Das erfindungsgemäße Abtrennverfahren kann aber auch an jeder anderen Stelle eines solchen Verbunds eingefügt werden.

In einer Ausführungsform der vorliegenden Erfindung wird der beim erfindungsgemäßen Abtrennverfahren eingesetzte C4-Kohlenwasserstoffstrom einer MTBE-Synthese nach der Abtrennung von MTBE entnommen und der butenhaltige Produktstrom anschließend einer 1-Butenabtrennung zugeführt, wonach sukzessive eine Oligomerisierung und eine oder mehrere Hydroformylierung(en) zur anschließenden Herstellung von 2-PH, ITDA und/oder INA erfolgen. Die einzelnen Verfahrensschritte können weitere Schritte wie beispielsweise die Abtrennung der Produkte oder die Aufarbeitung der resultierenden Ströme enthalten, sind hier jedoch nicht explizit genannt.

In einer weiteren Ausführungsform der vorliegenden Erfindung wird der beim erfindungsgemäßen Abtrennverfahren eingesetzte C4-Kohlenwasserstoffstrom einer 1-Butenabtrennung entnommen und der butenhaltige Produktstrom anschließend einer Oligomerisierung zugeführt, wonach eine oder mehrere Hydroformylierung(en) zur anschließenden Herstellung von 2-PH, ITDA und/oder INA erfolgen. Die einzelnen Verfahrensschritte können weitere Schritte wie beispielsweise die Abtrennung der Produkte oder die Aufarbeitung der resultierenden Ströme enthalten, sind hier jedoch nicht explizit genannt.

In einer weiteren Ausführungsform der vorliegenden Erfindung wird der beim erfindungsgemäßen Abtrennverfahren eingesetzte C4-Kohlenwasserstoffstrom einer Oligomerisierung entnommen und der butenhaltige Produktstrom anschließend einer Hydroformylierung zur anschließenden Herstellung von 2-PH zugeführt. Die einzelnen Verfahrensschritte können weitere Schritte wie beispielsweise die Abtrennung der Produkte oder die Aufarbeitung der resultierenden Ströme enthalten, sind hier jedoch nicht explizit genannt.

In einer weiteren Ausführungsform der vorliegenden Erfindung wird das erfindungsgemäße Abtrennverfahren am Ende des Verbunds eingesetzt. In dem Fall wird der eingesetzte C4-Kohlenwasserstoffstrom einer der Hydroformylierung sich anschließenden 2-PH-Herstellung entnommen. Der aus dem erfindungsgemäßen Abtrennverfahren sodann erhaltene butenhaltige Produktstrom kann in diesem Fall zurückgeführt werden und an einer geeigneten Stelle im Verbund, beispielsweise zur 1-Butenabtrennung, zur Oligomerisierung oder einer oder mehrere Hydroformylierung(en) eingesetzt werden. Dadurch kann die Effizienz des gesamten Verbunds gesteigert werden, da selbst nach Durchlaufen des letzten Verfahrensschrittes im Verbund noch bis zu 20 Gew.-% Butene vorhanden sein können.

Der butanhaltige Produktstrom kann unabhängig von der Stelle im Verbund, in der das erfindungsgemäße Abtrennverfahren angeordnet ist, beispielsweise einer adiabaten Oligomerisierung, einer Hydrierung der noch vorhandenen Butene oder einer n/-iso-Splittung der Butane, bei der n-Butan und Isobutan voneinander getrennt werden, zugeführt werden. Die n/iso-Splittung kann auch nach einer adiabaten Oligomerisierung erfolgen. Möglich wäre auch eine Einbindung des butanhaltigen Produktstroms vor der Oligomerisierung in einem oben beschriebenen Verbund aus MTBE-Synthese, 1-Butenabtrennung, Oligomerisierung und einer Hydroformylierung.

In einer besonders bevorzugten Ausführungsform der vorliegenden Erfindung kann die zur n/iso-Splittung erforderliche Energie zumindest teilweise durch Wärmeintegration mit der ersten Stufe einer zweistufigen Kondensation am Kopf des Desorbers erfolgen. Das hat den Vorteil, dass die Energie, die in der Kondensation anfällt, genutzt wird und nicht wie im Stand der Technik einfach an die Umgebung abgegeben wird.

Die vorliegende Erfindung wird nachfolgend anhand von Abbildungen erläutert. Die Abbildungen dienen der Veranschaulichung, sind aber nicht einschränkend zu verstehen.

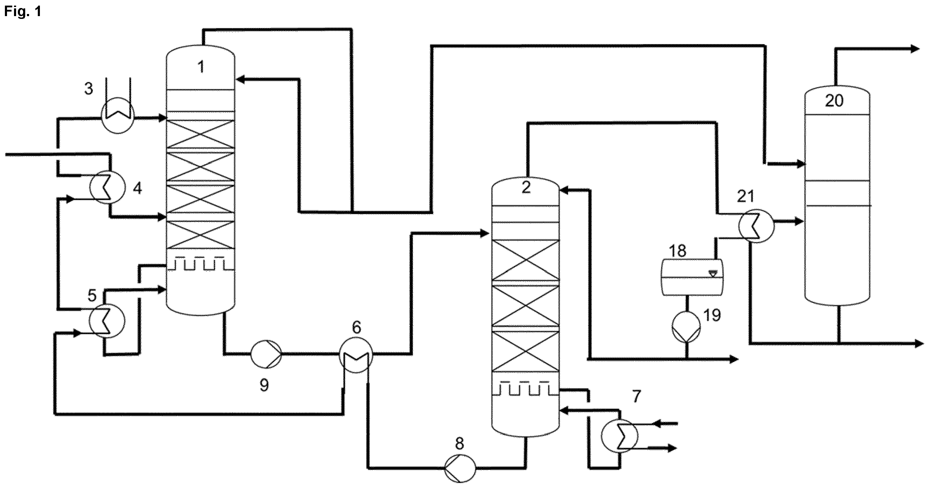

Fig. 1 zeigt die grundsätzliche Ausgestaltung der vorliegenden Erfindung. Der flüssige C4-Kohlenwasserstoffstrom wird über einen Wärmetauscher (4) verdampft und in den Absorber (1) geleitet. Das Lösemittel wird über einen Restkühler (3) - wenn notwendig - auf die gewünschte Temperatur gebracht und ebenfalls in den Absorber geleitet, wobei der Einlass räumlich gesehen oberhalb des Einlasses für den C4-Kohlenwasserstoffstrom ist, im vorliegenden Fall oberhalb des ersten Füllkörperbettes. Am Kopf des Absorbers (1) fällt der an Butanen angereicherte Strom an, der zum n/iso-Splitter (20) geführt wird. Vorher ist eine Kondensation möglich (nicht eingezeichnet). Zur Erwärmung des n/iso-Splitters wird die Kondensationswärme vom Kondensator (21) verwendet, der auch zweistufig ausgestaltet sein kann. Im Sumpf des Absorbers (1) wird das beladene Lösemittel gesammelt, was in der Abbildung durch den Kaminboden angedeutet ist. Dort wird zumindest ein Teil des beladenen Lösemittels abgenommen und über einen Absorberverdampfer (5) zum Sumpf des Absorbers (2) geführt. Aus dem Sumpf des Absorbers (1) wird das beladene Lösemittel abgenommen und mittels einer Pumpe (9) über den Wärmetauscher (6) zur Vorwärmung des beladenen Lösemittels zum Desorber (2) geleitet, wo die im Lösemittel vorhandenen Butene vom Lösemittel getrennt werden. Am Kopf des Desorbers fällt der an Butenen angereicherte Strom an. Dieser Strom wird einer Kondensation im Kondensator (21) geworfen und zu einem Phasentrenner (18) geleitet. Die wässrige Phase wird zum Desorber geführt. Der butenhaltige Produktstrom wird abgeführt. Lediglich ein möglicher Rückführstrom ist angedeutet. Im Sumpf des Desorbers (2) wird das zumindest teilweise von Butenen befreite Lösemittel gesammelt, was in der Abbildung durch den Kaminboden angedeutet ist. Dort wird zumindest ein Teil des beladenen Lösemittels abgenommen und über einen Desorberverdampfer (7) zum Sumpf des Desorbers geführt. Aus dem Sumpf des Desorbers (2) wird dann das von Butenen befreite Lösemittel abgenommen und mittels einer Pumpe (8) über den Wärmetauscher (6) zur Vorwärmung des beladenen Lösemittels, den Absorberverdampfer (5), den Wärmetauscher (4) zur Verdampfung des C4-Kohlenwasserstoffstroms und den Restkühler (3) zum Absorber zurückgeführt.

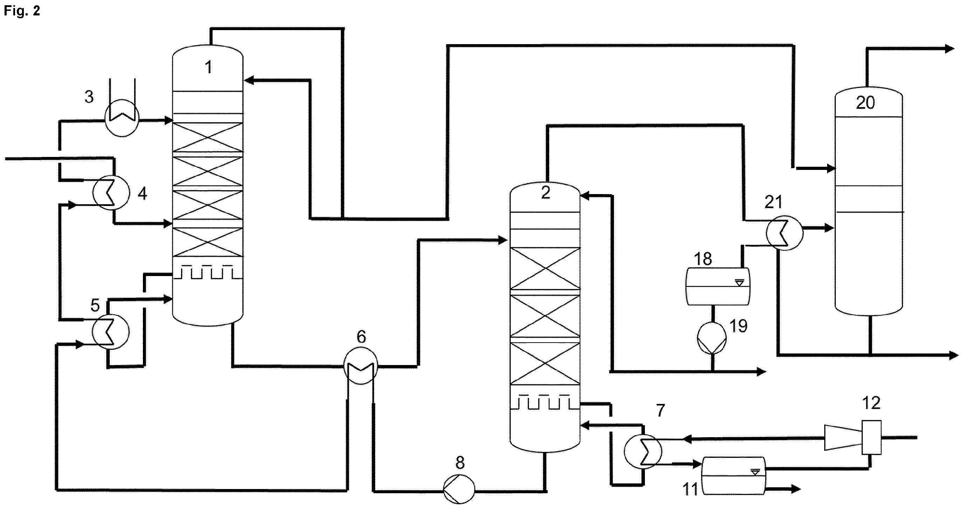

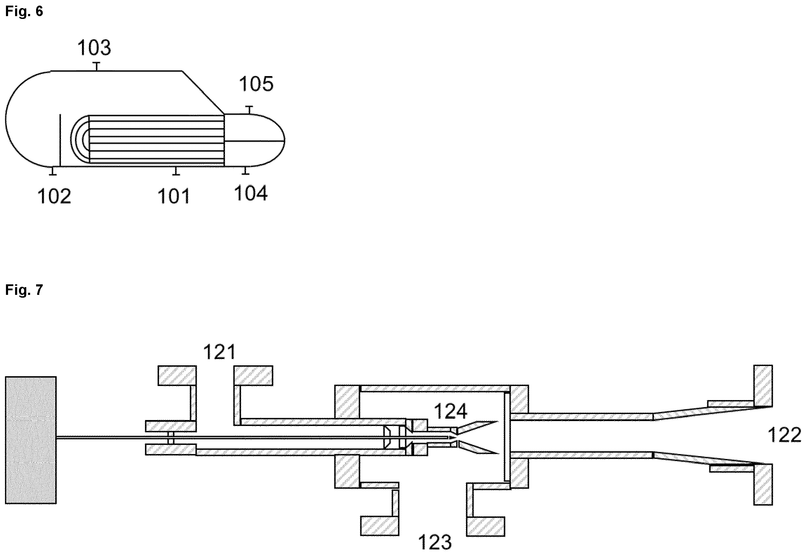

Fig. 2 zeigt eine weiterhin bevorzugte Ausführungsform der vorliegenden Erfindung, bei der am Desorberverdampfer (7) ein Dampfstrahler (12) vorhanden ist. Dieser Dampfstrahler wird mit dem regulären Heizdampf, also beispielsweise dem Mitteldruckdampf aus dem Dampfnetz, und dem Niederdruckdampf, der im Kondensatbehälter (11) anfällt, gespeist, wodurch ein Mischdampf entsteht, der dann als Heizdampf zum Desorberverdampfer (7) verwendet wird. Die Funktionsweise eines Dampfstrahlers wird bei der Beschreibung von Fig. 7 erläutert. Alles weitere entspricht den vorherigen Ausführungen zu Fig. 1.

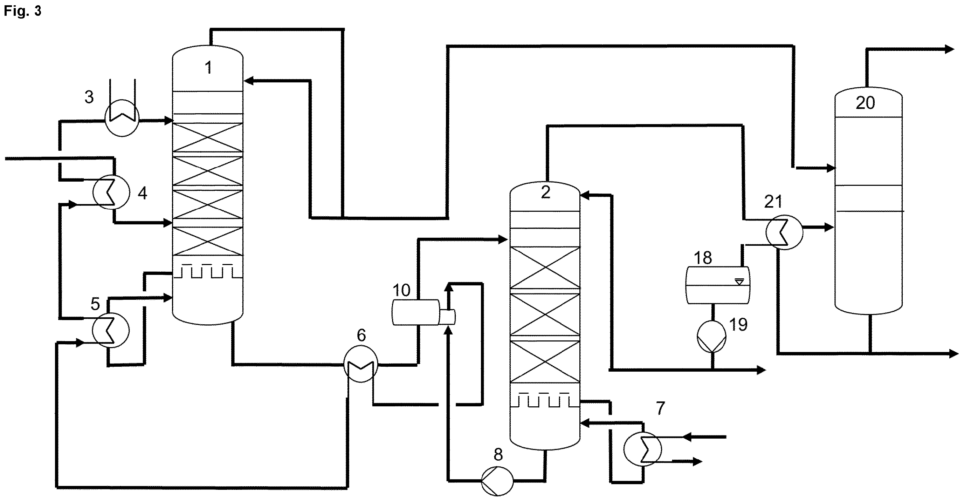

Fig. 3 zeigt eine bevorzugte Ausführungsform der vorliegenden Erfindung, bei der die Vorwärmung des zum Desorber (2) geführten beladenen Lösemittels zweistufig durchgeführt wird. Dabei findet die erste Wärmeübertragung am Wärmetauscher (6) und anschließend die zweite Wärmeübertragung im Kettleverdampfer (10) statt. Die Funktionsweise eines Kettleverdampfers wird bei der Beschreibung von Fig. 6 erläutert. Alles weitere entspricht den vorherigen Ausführungen zu Fig. 2.

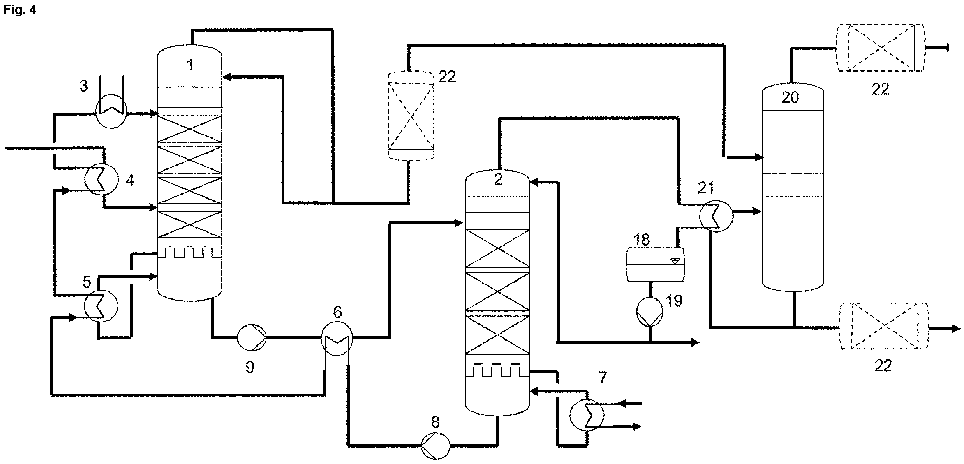

Fig. 4 zeigt eine bevorzugte Ausführungsform der vorliegenden Erfindung, bei der eine Hydrierung in einer Hydriereinheit (22) vorgesehen ist. Die Anordnung der Hydrierung ist variabel, weswegen die verschiedenen Möglichkeiten hier gestrichelt gezeichnet worden sind. Die Hydrierung kann dabei entweder vor dem Eintritt in den n/iso-Splitter oder nach dem n/iso-Splitter erfolgen. Nach dem n/iso-Splitter kann die Hydrierung am Kopfstrom und/oder am Sumpfstrom erfolgen. Alles weitere entspricht den vorherigen Ausführungen zu Fig. 1.

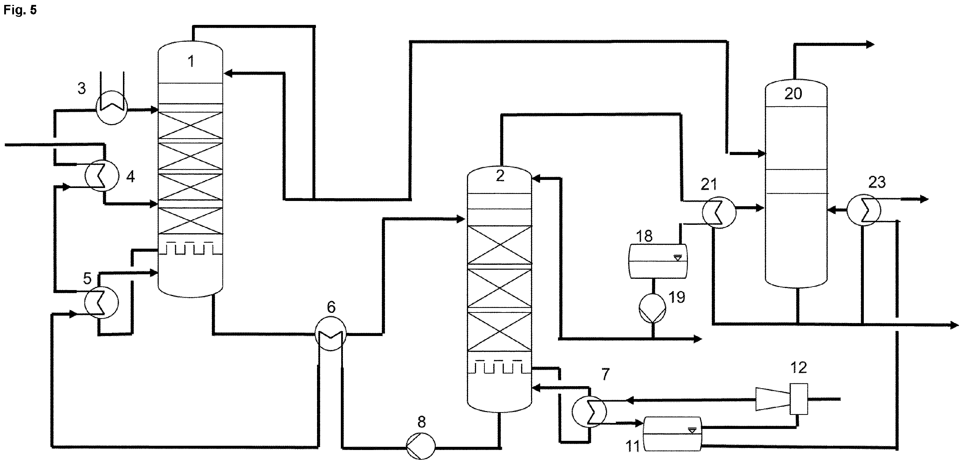

Fig. 5 zeigt eine bevorzugte Ausführungsform der vorliegenden Erfindung, bei die Wärme des Heißkondensats, das ebenfalls im Kondensatbehälter (11) anfällt, zusätzlich zur Beheizung des n/iso-Splitters (20) über den Wärmetauscher (23) verwendet wird. Alles weitere entspricht den vorherigen Ausführungen zu Fig. 2.

Fig. 6 zeigt den schematischen Aufbau eines Kettleverdampfers (10). Über den Feedstutzen (101) auf der Mantelseite wird der flüssige Feed in den Verdampfer gefahren. Der flüssige Feed wird teilweise im Kettleverdampfer verdampft und über den Gasstutzen (103) auf der Mantelseite in den Desorber gefahren. Der Anteil des Feeds, der nicht verdampft wird, läuft über ein Wehr ab und über den Produktstutzen (102) auf der Mantelseite als flüssiges Produkt in den Desorber (2). Auf der Rohrseite wird das heiße Sumpfprodukt des Desorbers (2) als Heizmedium verwendet, das über den Eintrittsstutzen (104) durch die Rohre gefahren wird und am Austrittsstutzen (105) wieder austritt.

Fig. 7 zeigt den schematischen Aufbau eines Dampfstrahlers (12). Der Treibdampf (121) ist hier der Heizdampf, insbesondere der Mitteldruckdampf aus dem Dampfnetz. Der Saugdampf (123) ist der Niederdruckdampf aus dem Kondensatbehälter. Beide werden über die Regeleinheit (124) vermischt und über den Ausgang als Mischdampf (122) zum Desorberverdampfer (7) geführt. Über die Regeleinheit können die Mengen von Treibdampf und Saugdampf eingestellt werden, wodurch Druck und Temperatur des Mischdampfes und damit die mögliche Heizleistung beeinflusst werden können.

### Beispiele

### Beispiel 1 - Ausführungsform gemäß Abbildung 1

Die in Abbildung 1 dargestellte Butan-Buten-Trennung wurde mit Aspen Plus v10 simuliert. Es wurde ein modifizierter NRTL-Parametersatz verwendet, um die Wechselwirkungen zwischen den Komponenten zu beschreiben. In der Simulation wurden 19 t/h eines kohlenwasserstoffhaltigen Feeds in eine Butan/Buten-Trennung (gemäß Abbildung 1) gefahren. Dieser enthält insgesamt 45 Gew.-% Butane (35 Gew.-% n- und 10 Gew.-% iso-Butan), 30 Gew.-% iso-Buten, 25 Gew.-% n-Buten besteht.

Insgesamt werden an dem Absorber 7 t/h an butanhaltiges Kopfprodukt entnommen. Das butanhaltige Kopfprodukt des Absorbers besteht aus 70 Gew.-% n-Butan, 27 Gew.-% iso-Butan und 3 Gew.-% Butene. Im Sumpf wird das mit dem butenhaltigen Produktstrom beladene Lösemittel entnommen. Der butenhaltige Produktstrom, enthält 14 Gew.-% n-Butan, 47 Gew.-% iso-Buten und 39 Gew.-% n-Buten. Mit dem eingestellten Lösemittel/Feed-Verhältnis von 13 kann demnach eine 98%ige Buten-Ausbeute erzielt werden. Der große Überschuss an Lösemittel wird dann auch für die Wärmeintegration verwendet.

Nur am Desorber (2) wird Energie von außerhalb der Anlage eingetragen, nämlich über den Heizdampf für den Desorberverdampfer (7). Es werden 6,9 MW an dem Desorberverdampfer (7) übertragen. Ansonsten wird in diesem Beispiel durchweg eine Wärmeintegration mit dem Lösemittel durchgeführt, sodass keine weitere externe Energie benötigt wird. Das heiße Sumpfprodukt des Desorbers (2) wird über den Wärmetauscher zur Vorwärmung (6) des Desorbers gefahren. Hier werden 8,4 MW übertragen. Nach der Vorwärmung des Desorbers (6) wird das Lösemittel zum Sumpfs des Absorbers (1) gefahren, wo 7,8 MW übertragen über den dortigen Wärmetauscher (5) übertragen werden. Zur Verdampfung des C4-Feedstroms werden vom Lösemittel weitere 1,8 MW über den Wärmetauscher (4) zur Feedverdampfung auf den Feed übertragen. In einem Restkühler (3) wird das Lösemittel mit Kühlwasser auf die optimale Extraktionstemperatur gebracht. Für dieses Beispiel wurden 40 °C angenommen. Dem Lösemittel müssen daher weitere 2,5 MW entzogen werden, um diese Temperatur zu erreichen. Durch diese Verschaltung kann der externe Wärmeeintrag reduziert und die interne Wärme optimal ausgenutzt werden. Wäre nämlich zumindest einer der Wärmetauscher (4), (5) und (6) nicht vorhanden, müssten die nicht übertragene Energie im Restkühler (3) entzogen und damit vernichtet werden. Außerdem müsste die dann nicht übertragene Energie extern zugeführt werden. Insgesamt werden bei dieser Verfahrensweise nur 6,9 MW externer Energie benötigt.

Zusätzlich wird der n/iso-Splitter mit der Kondensationswärme beheizt. Dadurch können 3,5 MW aus der Kondensation zur Auftrennung im n/iso-Splitter genutzt werden. Ist außerdem noch eine Hydrierung vorhanden, können weitere 0,7 MW aus der Hydrierung für die Auftrennung im n/iso-Splitter genutzt werden.

## Patentansprüche

1. Verfahren zur Abtrennung von Butenen aus einem C4-Kohlenwasserstoffstrom, der zumindest Butene und die Butane n-Butan und iso-Butan enthält, durch Extraktivdestillation mit einem Lösemittel, wobei das Verfahren die folgenden Schritte umfasst:
a. Zumindest teilweises Verdampfen des flüssigen C4-Kohlenwasserstoffstroms in einem Feedverdampfer, Zuführen des gasförmigen C4-Kohlenwasserstoffstroms und Zuführen des flüssigen Lösemittels oberhalb des C4-Kohlenwasserstoffstroms zu einem Absorber, in dem der C4-Kohlenwasserstoffstrom und das Lösemittel miteinander in Kontakt gebracht werden, wodurch überwiegend Butene aus dem C4-Kohlenwasserstoffstrom in das Lösemittel übergehen, wobei das so beladene Lösemittel in einem Flüssigkeitssammler des Absorbers gesammelt und durch einen Absorberverdampfer gefahren und dann unterhalb des Flüssigkeitssammlers in den Sumpf des Absorbers geleitet wird, wodurch überwiegend Butane aus dem beladenen Lösemittel ausgegast werden, und wobei das beladene Lösemittel anschließend als Sumpfstrom zu einem Desorber geführt wird und wobei am Kopf des Absorbers ein im Vergleich zum eingesetzten C4-Kohlenwasserstoffstrom an Butanen angereicherter Strom anfällt;
b. Zuführen des beladenen Lösemittels zu dem Desorber, in dessen Sumpf im Vergleich mit dem Sumpf des Absorber eine erhöhte Temperatur und bevorzugt ein geringerer Druck vorliegt und in dem die Butene vom Lösemittel getrennt werden, wodurch am Kopf des Desorbers ein an Butenen angereicherter Strom anfällt, wobei ein zumindest teilweise von Butenen befreite Lösemittel in einem Flüssigkeitssammler des Desorbers gesammelt, durch einen Desorberverdampfer gefahren und dann unterhalb des Flüssigkeitssammlers in den Sumpf des Desorbers geleitet wird, wodurch noch im Lösemittel vorhandene Butene ausgegast werden, und wobei das Lösemittel anschließend als Sumpfstrom zum Absorber zurückgeführt wird;
c. Zuführen des an Butanen angereicherten Stroms zu einem n/iso-Splitter, in dem n-Butan und iso-Butan voneinander getrennt werden, wodurch am Kopf des n/iso-Splitters ein an iso-Butan angereicherter Strom und im Sumpf des n/iso-Splitters ein an n-Butan angereicherter Strom anfällt;
**dadurch gekennzeichnet, dass** die Wärme des als Sumpfstrom des Desorbers abgenommen Lösemittels zur Wärmeintegration verwendet wird, in dem die Wärme des Lösemittels in jeweils mindestens einem Wärmetauscher für die Vorwärmung des zum Desorber geführten beladenen Lösemittels, zur Verdampfung im Absorberverdampfer und zur Verdampfung des flüssigen C4-Kohlenwasserstoffstroms eingesetzt wird, und dass
der an Butenen angereichter Strom am Kopf des Desorbers entnommen und einer Kondensation unterworfen wird, wobei die Kondensationswärme zumindest teilweise zur Beheizung des n/iso-Splitters verwendet wird.

2. Verfahren nach Anspruch 1, wobei das eingesetzte Lösemittel NMP ist.

3. Verfahren nach Anspruch 1 oder 2, wobei das Lösemittel bzw. das NMP Wasser enthält und der Wassergehalt zwischen 1 und 10 Gew.-%, vorzugsweise zwischen 4 und 9 Gew.-% liegt.

4. Verfahren nach einem der vorhergehenden Ansprüche, wobei der an Butenen angereicherte Strom, der am Kopf des Desorbers anfällt, zusätzlich Wasser enthält, welches aus dem Lösemittel stammt.

5. Verfahren nach Anspruch 5, wobei der an Butenen angereichter Strom am Kopf des Desorbers entnommen und einer Kondensation unterworfen wird, wobei ein wasserhaltiger Strom und ein butenhaltiger Produktstrom auskondensiert und voneinander getrennt werden.

6. Verfahren nach Anspruch 6, wobei der aus der Kondensation erhaltene butenhaltige Produktstrom einen Butengehalt von mindestens 70 Gew.-%, vorzugsweise mindestens 75 Gew.-%, besonders bevorzugt mindestens 86 Gew.-% bezogen auf die Gesamtzusammensetzung des butenhaltigen Produktstroms, aufweist.

7. Verfahren nach einem der vorhergehenden Ansprüche, wobei der an Butanen angereicherten Strom, der am Kopf des Absorbers abgenommen wird, vor dem Eintritt in den n/iso-Splitter einer Hydrierung unterworfen wird, um noch vorhandene Butene zu Butanen umzusetzen.

8. Verfahren nach Anspruch 7, wobei zumindest ein Teil der bei der Hydrierung entstehenden Reaktionswärme zur Beheizung des n/iso-Splitters verwendet wird.

9. Verfahren nach einem der Ansprüche 1 bis 6, wobei der am Kopf des n/iso-Splitters anfallende an iso-Butan angereicherte Strom und/oder der im Sumpf des n/iso-Splitters anfallende an n-Butan angereicherte Strom einer Hydrierung unterworfen werden, um noch vorhandene Butene zu Butanen umzusetzen.

10. Verfahren nach Anspruch 9, wobei zumindest ein Teil der bei der Hydrierung oder zumindest ein Teil der bei den beiden Hydrierungen entstehenden Reaktionswärme zur Beheizung des n/iso-Splitters verwendet wird.

11. Verfahren nach einem der vorhergehenden Ansprüche, wobei die Vorwärmung des zum Desorber geführten beladenen Lösemittels zweistufig durchgeführt wird, wobei eine erste eine Wärmeübertragung auf das Lösemittel in einem Wärmetauscher und eine zweite Wärmeübertragung auf das Lösemittel in einem Kettleverdampfer erfolgt.

12. Verfahren nach einem der vorhergehenden Ansprüche, wobei die Wärme zur Verdampfung im Desorberverdampfer durch Wärmeaustausch in einem Wärmetauscher mit einem geeigneten Wärmeträgermedium, insbesondere Heizdampf, eingetragen wird.

13. Verfahren nach einem der vorhergehenden Ansprüche, wobei der eingesetzte Heizdampf im Wärmetauscher zumindest teilweise kondensiert und dadurch ein Heißkondensat bei einem Druck von 10 bis 20 bar absolut, vorzugsweise 12 bis 17 bar absolut und einer Temperatur von 150 bis 200 °C, vorzugsweise 160 bis 190 °C anfällt, das zu einem Kondensatbehälter geleitet wird.

14. Verfahren nach Anspruch 13, wobei im Kondensatbehälter ein geringerer Druck vorliegt als im Wärmetauscher auf der Heizdampfseite und dadurch ein Teil des Heißkondensats wieder verdampft, wodurch der gesamte Dampf als Niederdruckdampf anfällt.

15. Verfahren nach einem der vorhergehenden Ansprüche, wobei der Heizdampf für den Desorberverdampfer mittels eines Dampfstrahlers, der mit Hochdruck- oder Mitteldruckdampf und dem im Kondensatbehälter anfallenden Niederdruckdampf gespeist wird, zur Verfügung gestellt wird.

## Claims

1. Process for separating butenes from a C4-hydrocarbon stream which contains at least butenes and the butanes n-butane and isobutane by extractive distillation with a solvent, wherein the process comprises the steps of:
a. at least partially evaporating the liquid C4-hydrocarbon stream in a feed evaporator, supplying the gaseous C4-hydrocarbon stream and supplying the liquid solvent above the C4-hydrocarbon stream to an absorber in which the C4-hydrocarbon stream and the solvent are contacted with one another to transfer predominantly butenes from the C4-hydrocarbon stream to the solvent, wherein the thus-laden solvent is collected in a liquid collector of the absorber, passed through an absorber evaporator and then passed into the bottom of the absorber below the liquid collector to outgas predominantly butanes from the laden solvent and wherein the laden solvent is subsequently passed to a desorber as bottoms stream and wherein a stream enriched in butanes relative to the employed C4-hydrocarbon stream is obtained at the top of the absorber;
b. supplying the laden solvent to the desorber, the bottom of which is at an elevated temperature and preferably a lower pressure relative to the bottom of the absorber and in which the butenes are separated from the solvent to obtain at the top of the desorber a stream enriched in butenes, wherein a solvent, at least partially freed of butenes, is collected in a liquid collector of the desorber, passed through a desorber evaporator and then passed into the bottom of the desorber below the liquid collector to outgas any butenes remaining in the solvent and wherein the solvent is subsequently recycled to the absorber as bottoms stream;
c. supplying the stream enriched in butanes to an n/iso splitter in which n-butane and isobutane are separated from one another to obtain a stream enriched in isobutane at the top of the n/iso splitter and a stream enriched in n-butane at the bottom of the n/iso splitter;
**characterized in that** the heat of the solvent withdrawn as a bottoms stream of the desorber is used for heat integration by employing the heat of the solvent in at least one respective heat exchanger for preheating the laden solvent passed to the desorber, for evaporation in the absorber evaporator and for evaporation of the liquid C4-hydrocarbon stream and **in that**
the stream enriched in butenes is withdrawn at the top of the desorber and subjected to a condensation, wherein the heat of condensation is at least partially used for heating the n/iso splitter.

2. Process according to Claim 1, wherein the employed solvent is NMP.

3. Process according to Claim 1 or 2, wherein the solvent/the NMP contains water and the water content is between 1% and 10% by weight, preferably between 4% and 9% by weight.

4. Process according to any of the preceding claims, wherein the stream enriched in butenes obtained at the top of the desorber additionally contains water originating from the solvent.

5. Process according to Claim 5, wherein the stream enriched in butenes is withdrawn at the top of the desorber and subjected to a condensation, wherein a water-containing stream and a butene-containing product stream are condensed out and separated from one another.

6. Process according to Claim 6, wherein the butene-containing product stream obtained from the condensation has a butene content of at least 70% by weight, preferably at least 75% by weight, particularly preferably at least 86% by weight, based on the total composition of the butene-containing product stream.

7. Process according to any of the preceding claims, wherein the stream enriched in butanes withdrawn at the top of the absorber is subjected to a hydrogenation before entry into the n/iso splitter to convert any remaining butenes into butanes.

8. Process according to Claim 7, wherein at least a portion of the heat of reaction formed during the hydrogenation is used for heating the n/iso splitter.

9. Process according to any of Claims 1 to 6, wherein the stream enriched in isobutane obtained at the top of the n/iso splitter and/or the stream enriched in n-butane obtained at the bottom of the n/iso splitter are/is subjected to a hydrogenation to convert any remaining butenes into butanes.

10. Process according to Claim 9, wherein at least a portion of the heat of reaction formed during the hydrogenation or at least a portion of the heat of reaction formed during the two hydrogenations is used for heating the n/iso splitter.

11. Process according to any of the preceding claims, wherein the preheating of the laden solvent passed to the desorber is performed in two stages, wherein a first heat transfer to the solvent is effected in a heat exchanger and a second heat transfer to the solvent is effected in a kettle evaporator.

12. Process according to any of the preceding claims, wherein the heat for evaporation in the desorber evaporator is introduced in a heat exchanger by heat transfer with a suitable heat transfer medium, in particular heating steam.

13. Process according to any of the preceding claims, wherein the employed heating steam undergoes at least partial condensation in the heat exchanger, thus generating a hot condensate at a pressure of 10 to 20 bar absolute, preferably 12 to 17 bar absolute, and a temperature of 150°C to 200°C, preferably 160°C to 190°C, which is passed to a condensate container.

14. Process according to Claim 13, wherein the pressure in the condensate container is lower than in the heating steam side of the heat exchanger, thus causing a portion of the hot condensate to be reevaporated, as a result of which the combined steam is obtained as low pressure steam.

15. Process according to any of the preceding claims, wherein the heating steam for the desorber evaporator is provided using a steam ejector supplied with high pressure or medium pressure steam and the low pressure steam obtained in the condensate container.

## Revendications

1. Procédé destiné à la séparation de butènes à partir d'un courant d'hydrocarbures en C4 qui contient au moins des butènes et les butanes n-butane et isobutane, par distillation extractive à l'aide d'un solvant, le procédé comprenant les étapes suivantes :
a. vaporisation au moins partielle du courant liquide d'hydrocarbures en C4 dans un évaporateur à simple passage, envoi du courant gazeux d'hydrocarbures en C4 et envoi du solvant liquide au-dessus du courant d'hydrocarbures en C4 à un absorbeur, dans lequel le courant d'hydrocarbures en C4 et le solvant sont mis en contact l'un avec l'autre, ce par quoi majoritairement des butènes passent du courant d'hydrocarbures en C4 dans le solvant, le solvant ainsi chargé étant recueilli dans un collecteur de liquide de l'absorbeur et conduit à travers un évaporateur d'absorbeur et ensuite envoyé au-dessous du collecteur de liquide dans la partie inférieure de l'absorbeur, ce par quoi majoritairement des butanes sont extraits sous forme de gaz du solvant chargé, et le solvant chargé étant ensuite envoyé en tant que courant de partie inférieure à un désorbeur et un courant enrichi en butanes en comparaison du courant d'hydrocarbures en C4 introduit apparaissant à la tête de l'absorbeur ;
b. envoi du solvant chargé au désorbeur, dans la partie inférieure duquel est présente une température élevée et de préférence est présente une pression plus faible, en comparaison de la partie inférieure de l'absorbeur, et dans lequel les butènes sont séparés du solvant, ce par quoi un courant enrichi en butènes apparaît à la tête du désorbeur, un solvant au moins en partie libéré de butènes étant recueilli dans un collecteur de liquide du désorbeur, dirigé à travers un évaporateur de désorbeur et ensuite conduit au-dessous du collecteur de liquide dans la partie inférieure du désorbeur, ce par quoi des butènes encore présents dans le solvant sont extraits sous forme de gaz, et le solvant étant ensuite renvoyé en tant que courant de partie inférieure à l'absorbeur ;
c. envoi du courant enrichi en butanes à un séparateur n/iso, dans lequel n-butane et isobutane sont séparés l'un de l'autre, ce par quoi un courant enrichi en isobutane apparaît à la tête du séparateur n/iso et un courant enrichi en n-butane apparaît dans la partie inférieure du séparateur n/iso ;
**caractérisé en ce que** la chaleur du solvant prélevé, en tant que courant de partie inférieure, du désorbeur est utilisée pour l'intégration de chaleur, la chaleur du solvant étant utilisée dans chaque fois au moins un échangeur thermique pour le préchauffage du solvant chargé envoyé au désorbeur, pour la vaporisation dans l'évaporateur d'absorbeur et pour la vaporisation du courant liquide d'hydrocarbures en C4, et **en ce que**
le courant enrichi en butènes est prélevé à la tête du désorbeur et soumis à une condensation, la chaleur de condensation étant utilisée au moins en partie pour le chauffage du séparateur n/iso.

2. Procédé selon la revendication 1, dans lequel le solvant utilisé est la NMP.

3. Procédé selon la revendication 1 ou 2, dans lequel le solvant ou la NMP contient de l'eau et la teneur en eau se situe entre 1 et 10 % en poids, de préférence entre 4 et 9 % en poids.

4. Procédé selon l'une quelconque des revendications précédentes, dans lequel le courant enrichi en butènes, qui apparaît à la tête du désorbeur, contient en outre de l'eau qui provient du solvant.

5. Procédé selon la revendication 5, dans lequel le courant enrichi en butènes est prélevé à la tête du désorbeur et soumis à une condensation, où un courant contenant de l'eau et un courant de produit contenant des butènes sont condensés et séparés l'un de l'autre.

6. Procédé selon la revendication 6, dans lequel le courant de produit contenant des butènes, obtenu à partir de la condensation, présente une teneur en butènes d'au moins 70 % en poids, de préférence au moins 75 % en poids, de façon particulièrement préférée au moins 86 % en poids, par rapport à la composition totale du courant de produit contenant des butènes.

7. Procédé selon l'une quelconque des revendications précédentes, dans lequel le courant enrichi en butanes, qui est prélevé à la tête de l'absorbeur, est soumis à une hydrogénation avant l'entrée dans le séparateur n/iso, afin de convertir en butanes les butènes encore présents.

8. Procédé selon la revendication 7, dans lequel au moins une partie de la chaleur de réaction produite lors de l'hydrogénation est utilisée pour le chauffage du séparateur n/iso.

9. Procédé selon l'une quelconque des revendications 1 à 6, dans lequel le courant enrichi en isobutane, apparaissant à la tête du séparateur n/iso et/ou le courant enrichi en n-butane, apparaissant dans la partie inférieure du séparateur n/iso, est/sont soumis à une hydrogénation, afin de convertir en butanes les butènes encore présents.

10. Procédé selon la revendication 9, dans lequel au moins une partie de la chaleur de réaction produite lors de l'hydrogénation ou au moins une partie des chaleurs de réaction produites lors des deux hydrogénations est utilisée pour le chauffage du séparateur n/iso.

11. Procédé selon l'une quelconque des revendications précédentes, dans lequel le préchauffage du solvant chargé, envoyé au désorbeur, est effectué en deux étapes, une première effectuant un transfert de chaleur au solvant dans un échangeur thermique et une deuxième effectuant un transfert de chaleur au solvant dans un évaporateur à faisceau de tubes.

12. Procédé selon l'une quelconque des revendications précédentes, dans lequel la chaleur destinée à la vaporisation dans l'évaporateur de désorbeur est introduite par échange de chaleur dans un échangeur thermique à l'aide d'un fluide caloporteur approprié, en particulier de la vapeur de chauffage.

13. Procédé selon l'une quelconque des revendications précédentes, dans lequel la vapeur de chauffage utilisée dans l'échangeur thermique est au moins en partie condensée et il apparaît ainsi un condensat chaud sous une pression de 10 à 20 bars absolus, de préférence 12 à 17 bars absolus et à une température de 150 à 200 °C, de préférence 160 à 190 °C, qui est envoyé à un récipient de condensat.

14. Procédé selon la revendication 13, dans lequel dans le récipient de condensat est présente une pression plus faible que dans l'échangeur thermique du côté vapeur de chauffage et une partie du condensat chaud est ainsi vaporisée de nouveau, ce par quoi la vapeur totale apparaît sous forme de vapeur sous basse pression.

15. Procédé selon l'une quelconque des revendications précédentes, dans lequel la vapeur de chauffage pour l'évaporateur de désorbeur est mise à disposition au moyen d'un injecteur de vapeur qui est alimenté avec de la vapeur sous haute pression ou moyenne pression et la vapeur sous basse pression apparaissant dans le récipient de condensat.
